**Europäisches Patentamt**

(19) **European Patent Office**

**Office européen des brevets**

(11) Veröffentlichungsnummer: **0 269 946**
**B1**

(12) # EUROPÄISCHE PATENTSCHRIFT

(45) Veröffentlichungstag der Patentschrift:
**18.04.90**

(21) Anmeldenummer: **87116938.9**

(22) Anmeldetag: **17.11.87**

(51) Int. Cl.⁴: **A61F 5/01**, A61F 5/04

(54) **Sprunggelenkstütze.**

(30) Priorität: **29.11.86 DE 3640915**

(43) Veröffentlichungstag der Anmeldung:
**08.06.88 Patentblatt 88/23**

(45) Bekanntmachung des Hinweises auf die Patenterteilung:
**18.04.90 Patentblatt 90/16**

(84) Benannte Vertragsstaaten:
**AT DE FR GB IT NL SE**

(56) Entgegenhaltungen:
**CH-A- 607 614**
**DE-A- 3 435 955**
**DE-C- 272 865**
**GB-A- 1 515 153**
**US-A- 2 816 541**

(73) Patentinhaber: **Hefele, Wilhelm Josef,**
**Breitenbergstrasse 3, D-8955 Aitrang(DE)**

(72) Erfinder: **Hefele, Wilhelm Josef, Breitenbergstrasse 3,**
**D-8955 Aitrang(DE)**

(74) Vertreter: **Hübner, Hans–Jürgen, Dipl.-Ing.,**
**Mozartstrasse 31, D-8960 Kempten/Allgäu(DE)**

## Beschreibung

Die Erfindung betrifft eine Stützeinrichtung für das Sprunggelenk von Patienten, mit einem, aus steifem, dünnem Material bestehenden U-förmigem Bügel, der den Fuß des Patienten untergreift, wobei die Bügelschenkel seitlich an der Wade anliegend bis über den Knöchelbereich nach oben stehen und mit einem Verbindungsmittel, das als Lagesicherung für die Bügelschenkel diese eng umschließt.

Eine derartige Stützeinrichtung ist aus der DE-A-34 35 955 bekannt. Der U-förmige Bügel besteht aus Niedertemperatur-Thermoplast und kann durch Wärmeanwendung in gewissem Umfang verformt werden. Das Verbindungsmittel ist eine elastische Binde, die um die Wade des Patienten unter Einschluß der relativ kurzen Schenkel des U-Bügels herumgewickelt wird. Diese bekannte Stützeinrichtung kann zur weiteren Sicherheit nach Abnahme eines Gehgipsverbandes durchaus verwendet werden, sie kann jedoch den Gehgipsverband nach der operativen Versorgung nicht ersetzen. Der bis heute übliche Gehgipsverband hat aber die bekannten Nachteile der notwendigen Materialdicke, des erheblichen Gewichtes und der mangelnden Hygiene. Der Patient kann keine Konfektionsschuhe tragen. Außerdem ergibt sich ein vorübergehender Muskelschwund.

Die GB-A 1 515 153 zeigt eine weiche biegsame Hülse aus elastischem Schaummaterial in Form eines Zuschnittes, der im Knöchelbereich oberhalb der Ristöffnung und der Fersenöffnung verklebt ist oder aus einem einstückigen Formkörper besteht. In einem Seitenbereich der Hülse ist innenseitig eine elastische Schaumeinlage mit Nylon-Rücken zur Erhöhung der Druckfestigkeit vorgesehen. Eine solche Schaumstoffhülse kann allenfalls Druckbeanspruchungen auf den Knöchelbereich lindern, jedoch keinesfalls das Sprunggelenk unbeweglich fixieren.

Die US-A 2 816 541 schlägt eine Stützvorrichtung für das Schienenbein vor. Auch hier wird eine elastische Hülse verwendet, die über den Fuß gestreift wird und im Vorderbereich zwei nach oben vorstehende biegsame Schienen aufweist. Eine Wickelbinde hält die Vorrichtung in enger Anlage am Bein. Für das Schienenbein wird ein gewisser Schutz erzielt, jedoch ist diese Vorrichtung ebenfalls nicht als Sprunggelenkstütze einzusetzen.

Die DE-A 272 865 zeigt eine Sprunggelenkstütze, bei der von einer Sohlenplatte zwei seitliche Lappen unterschiedlicher Länge nach oben stehen, deren eine eine Stahldrahtfedereinlage aufweist. Beide Seitenlappen werden mittels eines Bindemittels am Bein festgeschnallt. Zwar läßt sich auch nach diesem Vorschlag ein gewisser Stützeffekt realisieren, jedoch ist eine echte Fixation des Sprunggelenkes nicht möglich.

Schließlich ist aus der CH-A 607 614 eine Klebebandage bekannt, die den Sohlenbereich abdeckt und mittels dreier Lappenpaare am Fuß bzw. Bein befestigt wird. Die Lappen jedes Lappenpaares umschließen den Fuß im Zehenbereich, oberhalb des Ristes und oberhalb des Knöchels. Die Lappen sind miteinander verklebt. Allein schon deswegen, weil das Material dieser Bandage nachgiebig ist, läßt sich diese Bandage nicht für den erfindungsgemäßen Zweck der Fixation des Sprunggelenkes einsetzen.

Aufgabe der Erfindung ist es, die Sprunggelenkstütze der eingangs genannten Art dahingehend zu verbessern, daß sie anstelle eines Gehgipsverbandes nach der Operation des Sprunggelenkes oder bei konservativer Behandlung nach Abschwellen des Ödemes anstelle eines Gehgipses verwendet werden kann, um das Sprunggelenk zu fixieren.

Diese Aufgabe wird erfindungsgemäß dadurch gelöst, daß der Bügel eine mit den Schenkeln einstückige, oberhalb der Ristöffnung beginnende starre Vorderwand sowie an den oberen Enden der Schenkel nach hinten anschließende und mit diesen einstückige steife Lappen aufweist, deren Enden einander im Bereich der Achillessehne wenigstens nahezu berühren und daß das Verbindungsmittel aus einem zweiteiligen Verschluß besteht, dessen Teile außen an den Schenkeln und/oder den Lappen befestigt sind.

Während die bekannte Stützeinrichtung aus einem vorn und hinten offenen U-Bügel besteht, der lediglich mittels einer Elastikbinde an der Wade befestigt wird, besteht die erfindungsgemäße Stützeinrichtung aus einem geschlossenen Körper, der einen ungleich höheren Widerstand gegen Verwindung und Verbiegung aufweist. Die beiden Schenkel können dank der sie verbindenden Vorderwand nicht relativ zueinander verschoben werden. Das Schienenbein liegt an der Vorderwand der Stützeinrichtung an. Die während der Gangphase auftretenden Kräfte werden vom causalen Schienenanteil aufgefangen und über die kollateralen Anteile auf den distalen Unterschenkelbereich übertragen. Eine Bewegung des Sprunggelenkes wird ausgeschlossen. Bei der eingangs genannten Stützrichtung hat demgegenüber die Elastikbinde eine viel zu große Elastizität, die beim Gehen des Patienten eine Kraftübertragung auf das Sprunggelenk nicht ausschließt. Dank der Erfindung sind hypervarisierende bzw. -valgisierende Bewegungen im Fersenbereich, die aus medizinischer Sicht unbedingt zu vermeiden sind, bei angelegter Stützeinrichtung unmöglich. Die neuartige Stützeinrichtung fixiert also das Sprunggelenk wie ein Gehgipsverband, hat aber nicht dessen Nachteile. Der Patient kann duschen, baden und schwimmen und seine herkömmlichen Schuhe tragen.

Vorteilhafte Ausgestaltungen bestehen darin, daß das Joch des Bügels nach hinten verlängert ist und eine Fersenauflage bildet und daß die Schenkel vom Knöchelbereich abwärts nach hinten zunehmend verlängert sind und seitliche Fersenstützflächen bilden. Die neue Stützeinrichtung ist vorzugsweise als Gießstück mit Verstärkungseinlage ausgebildet. Dank der Verstärkungseinlage bildet die Stützeinrichtung eine absolut formstabile Schale. Im Wadenbereich ist diese Schale ringsum geschlossen. Die beiden Lappen, die gegenüber dem übrigen Umfangsteil der Schale eine gewisse Elastizität haben, um die Einstiegöffnung freilegen zu können, stützen sich in der geschlossenen Stellung mit ihren Hinterkanten aneinander ab, sodaß der Verschluß nur die Aufgabe hat, die Lappen in dieser Stellung zu sichern. Als Verschluß kommt ein Klettverschluß, eine Schnallenverschluß oder ein ähnlicher ansich bekannter Verschluß infrage.

Im Bereich der Ränder der Ristöffnung, der Einstiegöffnung und der oberen Wadenöffnung fehlt die Verstärkungseinlage, sodaß diese Bereiche elastischer ausgebildet sind und sich dem Fuß des Patienten anschmiegen können.

Das Material der neuen Stützeinrichtung weist vorzugsweise eine Außenlage aus mehrschichtigem Perlongewebe, eine Innenschicht aus Perlongewebe und eine Zwischenschicht aus einer Kohlefasermatte auf, wobei alle Schichten durch ein Polyacryl-Gießharz zusammenlaminiert sind. Im Knöchel- und im Schienenbeinbereich befindet sich innenseitig eine Polsterung und es ist vorzugsweise weiterhin vorgesehen, daß die gesamte Innenfläche der Stützeinrichtung mittels einer wasserdichten Futterauflage versehen ist, die an den Rändern der Ristöffnung, der Einstiegöffnung und der Wadenöffnung nach außen umgelegt ist.

Dank der Herstellung der Stützeinrichtung in einem kombinierten Gieß-und Laminierverfahren ist eine genaue anatomische Anpassung an ein Fußmodell möglich.

Anhand der Zeichnung, die ein Ausführungsbeispiel darstellt, sei die Erfindung näher beschrieben. Es zeigt

FIGUR 1 eine perspektivische Ansicht einer an einem Fuß angelegten Sprunggelenkstütze,

FIGUR 2 eine perspektivische Ansicht der Sprunggelenkstütze ohne Verschluß, jedoch mit Veranschaulichung der Verstärkungsbereiche und

FIGUR 3 eine vertikale Querschnittansicht der Sprunggelenkstütze.

Die mit 10 bezeichnete Stützeinrichtung besteht aus einem im Querschnitt U-förmigen Bügel 12, mit einem Joch 14, das eine Fußauflage bildet und zwei Schenkeln 16, die die Seitenwände der Stützeinrichtung 10 bilden. Die beiden Schenkel 16 sind einstückig mit einer Vorderwand 18 zu einem geschlossenen nahtlosen Hohlkörper miteinander verbunden. Die Schenkel 16 und die Vorderwand 18 sind bis weit über den Knöchelbereich nach oben geführt und enden etwa auf gleichem Niveau an der Wadenöffnung 20. Die Vorderwand 18 erstreckt sich bis zum Rist des Fußes herab und umschließt zusammen mit den Schenkeln 16 und dem Joch 14 eine Ristöffnung 22. Im oberen Bereich der Stützeinrichtung 10 schließen sich an beide Schenkel 16 nach hinten weisende gewölbte Lappen 24 an, deren Endkanten 26 im geschlossenen Zustand der Stützeinrichtung aneinander abgestützt sind, sodaß in diesem Bereich die Wade des Patienten ringsum geschlossen ist.

Das Joch 14 ist nach hinten verlängert, sodaß eine Fersenauflagefläche 28 gebildet ist. Die beiden Schenkel 16 sind unterhalb des Knöchelbereiches ebenfalls nach unten allmählich zunehmend verlängert, wodurch Seitenstützflächen 30 für die Ferse gebildet werden. Die beiden Hinterkanten der Schenkel 16, die Unterkanten der Lappen 24 und die Fersenauflagefläche 28 des Joches 14 begrenzen eine rückwärtige Öffnung 32, die nach elastischem Auswärtsziehen der Lappen 24 eine Einstiegöffnung bildet. Nach dem Einstieg des Patienten schnappen die Lappen 24 um die Wade. Die Endkanten 26 der Lappen 24 stützen sich dabei aneinander ab und werden in dieser Lage durch eine Verschlußeinrichtung 34 gesichert, die hier als Klettbandverschluß dargestellt ist.

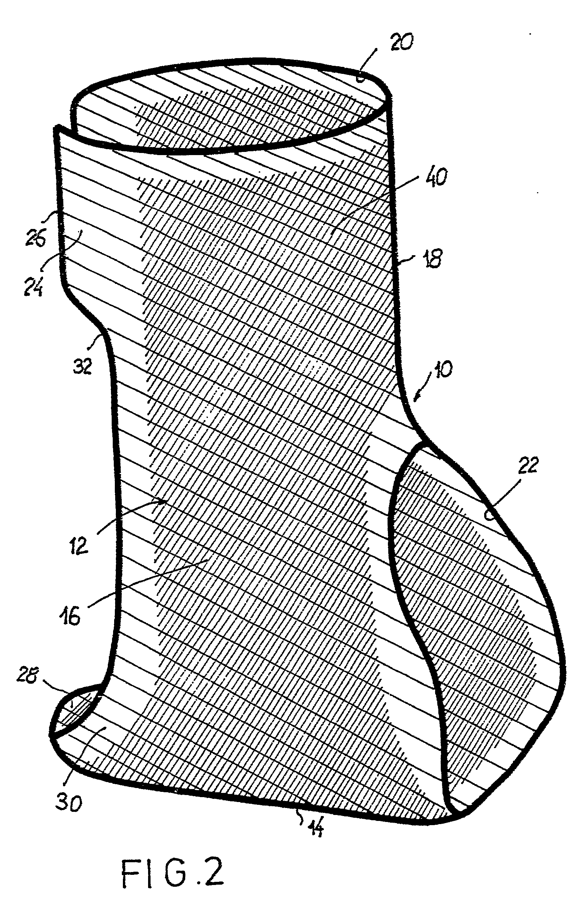

Die Stützeinrichtung 10 besteht aus einem Mehrlagenmaterial. Die Innenlage 36 und die Außenlage 38 bestehen je aus mehreren Schichten eines Perlongewebes. Zwischen diesen beiden Lagen 36, 38 befindet sich eine Verstärkungseinlage 40 aus einer Kohlefasermatte. Diese Verstärkungseinlage 40 erstreckt sich im Hauptbereich der Stützeinrichtung 10 und umfaßt insbesondere die Schenkel 16, das Joch 14 und die Vorderwand 18. In FIGUR 2 ist die Verstärkungseinlage doppelt schraffiert dargestellt. Die Verstärkungseinlage 40 erstreckt sich nicht im Bereich der Lappen 24 und längs der Ränder, die die Ristöffnung 22, die Wadenöffnung 20 und die Einstiegöffnung 32 begrenzen. Im Bereich der Verstärkungseinlage 40 ist die Stützeinrichtung 10 absolut starr und formstabil. Die nicht verstärkten Bereiche der Stützeinrichtung 10 sind dagegen flexibel, sodaß diese Bereich sich dem Fuß des Patienten anschmiegen können.

Die Innenlagen 36, 38 und die Verstärkungseinlage 40 sind durchgehend mit einem Polyacryl-Gießharz getränkt.

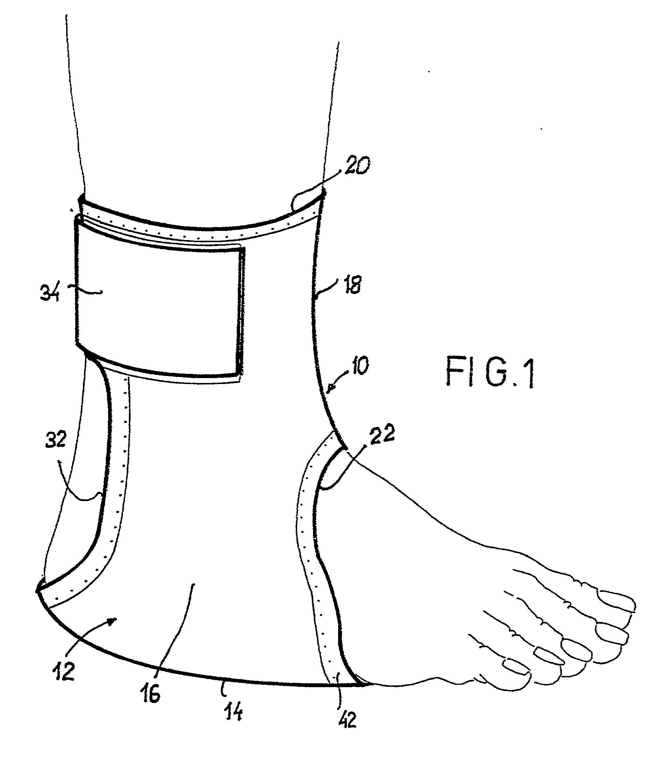

Im Knöchel-und Schienbeinbereich befindet sich innenseitig auf der Innenlage 36 eine nicht dargestellte Polsterauflage. Die gesamte Innenfläche der Stützeinrichtung 10 ist mittels eines wasserdichten Futters 42 abgedeckt, das an den Öffnungen 20, 22, 32 der Stützeinrichtung 10 herausgeführt und nach außen umgelegt und anschließend vernäht ist, wie in FIGUR 1 veranschaulicht ist.

Zusammenfassend sei festgestellt, daß der Bügel 12 mit Vorderwand 18 und Lappen 24 eine einstückige nahtlose Schale bilden, die im Bereich der Verstärkung 40 absolut form- und verwindungssteif ist, wobei die Schale dank der sich an den Endkanten 26 aneinander abstützender Lappen 24 vollkommen um die Wade des Patienten herum geschlossen ist. Der Verschluß 34 hat dabei keine tragende Funktion sondern dient lediglich der Lagesicherung der Lappen 24.

Das kombinierte Laminier-Gießverfahren der Stützeinrichtung 10 erlaubt eine genaue Anpassung an die Anatomie des Fußes des Patienten unter Verwendung eines Fußmodells. Dabei wird das Joch 14 des Bügels 12 entsprechend geformt, um eine mediale Längsgewölbestütze für den Fuß zu bilden.

## Patentansprüche

1. Stützeinrichtung für das Sprunggelenk von Patienten, mit einem aus steifem, dünnem Material bestehenden U-förmigem Bügel (12), der den Fuß des Patienten untergreift, wobei die Bügelschenkel (16) seitlich an der Wade anliegend bis über den Knöchelbereich nach oben stehen und mit einem Verbindungsmittel (34), das als Lagesicherung für die Bügelschenkel (16) diese eng umschließt, dadurch gekennzeichnet, daß der Bügel (12) eine mit den Schenkeln (16) einstückige, oberhalb der Ristöffnung (22) beginnende starre Vorderwand (18) sowie an den oberen Enden der Schenkel (16) nach hinten anschließende und mit diesen einstückige steife Lappen (24) aufweist, deren Enden einander im Bereich der Achillessehne wenigstens nahezu berühren und daß das Verbindungsmittel aus einem zweiteiligen Verschluß (34) besteht, dessen Teile außen an den Schenkeln (16) und/oder den Lappen (24) befestigt sind.

2. Stützeinrichtung nach Anspruch 1, dadurch gekennzeichnet, daß das Joch (14) des Bügels (12) nach hinten verlängert ist und eine Fersenauflage (28) bildet.

3. Stützeinrichtung nach Anspruch 2, dadurch gekennzeichnet, daß die Schenkel (16) vom Knöchelbereich abwärts nach hinten zunehmend verlängert sind und seitliche Fersenstützflächen (30) bilden.

4. Stützeinrichtung nach einem der Ansprüche 1 bis 3, dadurch gekennzeichnet, daß sie als Gießstück mit Verstärkungseinlage (40) ausgebildet ist.

5. Stützeinrichtung nach einem der Ansprüche 1 bis 4, dadurch gekennzeichnet, daß sie im Bereich der Ränder der Ristöffnung (22), der Einstiegöffnung (32) und der oberen Wadenöffnung (20) elastischer ausgebildet ist als in den dazwischenliegenden Bereichen.

6. Stützeinrichtung nach Anspruch 4 oder 5, dadurch gekennzeichnet, daß die Verstärkungseinlage (40) aus einem starren Körper besteht, der die größten Teile des Joches (14) , der Vorderwand (18) und beider Schenkel (12), jedoch mit Ausnahme der Lappen (24) umfaßt.

7. Stützeinrichtung nach Anspruch 4 oder 6, dadurch gekennzeichnet, daß die Verstärkungseinlage (40) eine Kohlefasermatte aufweist.

8. Stützeinrichtung nach einem der Ansprüche 1 bis 7, dadurch gekennzeichnet, daß das Material im Bereich der Verstärkungseinlage (40) eine Außenlage (38) aus mehrschichtigem Perlongewebe, eine Innenlage (36) gleichen Aufbaus und eine Zwischenlage aus einer Kohlefasermatte aufweist und daß alle Lagen (36, 38, 40) durch eine Polyacryl-Gießharzmasse zusammenlaminiert sind.

9. Stützeinrichtung nach einem der Ansprüche 1 bis 8, dadurch gekennzeichnet, daß im Knöchel- und Schienbeinbereich innenseitig eine Polsterung vorgesehen ist.

10. Stützeinrichtung nach einem der Ansprüche 1 bis 9, dadurch gekennzeichnet, daß ihre gesamte Innenfläche mittels einer wasserdichten Futterauflage (42) versehen ist, die an den Rändern der Ristöffnung (22), der Einstiegöffnung (32) und der Wadenöffnung (20) nach außen umgelegt ist.

**Revendications**

1. Dispositif de support pour l'articulation du pied de patients, à bride (12) en forme de U en un matériau fin et rigide, passant sous le pied du patient, les branches (16) de la bride s'étendant vers le haut jusqu'au-dessus de la zone de cheville, en s'appuyant latéralement contre le mollet, et à moyen de connexion (34) entourant étroitement les branches (16) de la bride afin de les maintenir en place, caractérisé en ce que la bride (12) présente une paroi frontale (18) rigide qui commence au-dessus de l'ouverture de cou-de-pied (22) et forme une seule pièce avec les branches (16) ainsi que des bandes (24) rigides attachées vers l'arrière aux extrémités supérieures des branches (16) et formant une seule pièce avec ces dernières, dont les extrémités entrent au moins presque en contact l'une avec l'autre à l'endroit du tendon d'Achille et que le moyen de connexion consiste en une fermeture (34) en deux pièces dont les pièces sont fixées à l'extérieur aux branches (16) et/ou aux bandes (24).

2. Dispositif de support suivant la revendication 1, caractérisé en ce que la travée (14) de la bride (12) se prolonge vers l'arrière et forme un appui de talon (28).

3. Dispositif de support suivant la revendication 2, caractérisé en ce que les branches (16) se prolongent, à partir de la zone de la cheville, vers le bas et vers l'arrière en s'évasant et forment des surfaces d'appui latérales de talon (30).

4. Dispositif de support suivant l'une des revendications 1 à 3, caractérisé en ce qu'il est formé comme une pièce coulée à couche de renforcement (40).

5. Dispositif de support suivant l'une des revendications 1 à 4, caractérisé en ce que, à l'endroit des bords de l'ouverture de cou-de-pied (22), de l'ouverture de pénétration du pied (32) et de l'ouverture de mollet supérieure (20), il est formé de manière plus élastique qu'aux endroits situés entre ceux-ci.

6. Dispositif de support suivant la revendication 4 ou 5, caractérisé en ce que la couche de renforcement (40) consiste en un corps rigide comprenant la majeure partie de la travée (14), de la paroi frontale (18) et des deux branches (12), toutefois avec exception des bandes (24).

7. Dispositif de support suivant la revendication 4 ou 6, caractérisé en ce que la couche de renforcement (40) présente une natte de fibres de carbone.

8. Dispositif de support suivant l'une des revendications 1 à 7, caractérisé en ce que le matériau présente, à l'endroit de la couche de renforcement (40), une couche extérieure (38) en tissu perlon à plis multiples, une couche intérieure (36) de même construction et une couche intermédiaire réalisée en une natte de fibres de carbone et que toutes les couches (36, 38, 40) sont laminées entre elles par une masse de résine coulée polyacrylique.

9. Dispositif de support suivant l'une des revendications 1 à 8, caractérisé en ce que, à l'endroit de la cheville et du tibia, il est prévu un rembourrage du côté intérieur.

10. Dispositif de support suivant l'une des revendications 1 à 9, caractérisé en ce que toute sa surface intérieure est munie d'une couche de doublure imperméable (42) repliée vers l'extérieur aux bords de l'ouverture de cou-de-pied (22), de l'ouverture de pénétration du pied (32) et de l'ouverture de mollet (20).

**Claims**

1. A support device for the ankle joint of patients, having a U-shaped strap (12) which is made of a stiff, thin material and grips the patients's foot below, the strap legs (16) projecting upwards to beyond the ankle area whilst laterally abutting against the calf, and having a connecting device (34) which as a device to secure the position of the strap legs (16) tightly encloses the latter, characterized in that the strap (12) has both a rigid front wall (18) which is in one piece with the legs (16) and begins above the instep opening (22), and stiff flaps (24) which are connected to the upper ends of the legs (16), to the rear, and are in one piece with the latter, the ends of these flaps almost touching one another, at least in the area of the Achilles tendon, and in that the connecting device is composed of a two-part fastening (34) whose parts are secured to the outside of the legs (16) and/or the flaps (24).

2. A support device in accordance with Claim 1, characterized in that the yoke (14) of the strap (12) is extended to the rear and forms a heel rest (28).

3. A support device in accordance with Claim 2, characterized in that from the ankle area downwards and to the rear the legs (16) are increasingly extended and form lateral heel-support-surfaces (30).

4. A support device in accordance with any one of Claims 1 to 3, characterized in that it is designed as a cast part with a reinforcing insert (40).

5. A support device in accordance with any one of Claims 1 to 4, characterized in that it is more flexible in design in the edge areas of the instep opening (22), of the entrance opening (32) and of the upper calf opening (20) than in the areas in between.

6. A support device in accordance with Claim 4 or 5, characterized in that the reinforcing insert (40) is composed of a rigid body which encloses the majority of the yoke (14), of the front wall (18) and of both legs (12), with the exception however of the flaps (24).

7. A support device in accordance with Claim 4 or 6, characterized in that the reinforcing insert (40) has a charcoal-fibre matting.

8. A support device in accordance with any one of Claims 1 to 7, characterized in that in the area of the reinforcing insert (40) the material has an outer layer (38) made of a multilayer Perlon fabric, an inner layer (36) of the same construction and an intermediate layer made out of a charcoal-fibre matting, and in that all the layers (36, 38, 40) are laminated together by means of a polyacryl casting-resin-substance.

9. A support device in accordance with any one of Claims 1 to 8, characterized in that there is padding on the inside in the ankle and shin areas,

10. A support device in accordance with any one of Claims 1 to 9, characterized in that its whole inner surface is provided by means of a watertight lining-support (42) which is put around the edges of the instep opening (22), of the entrance opening (32) and of the calf opening (20), in an outward direction.

FIG.1

FIG.2